# EUROPEAN PATENT SPECIFICATION

(11) **EP 4 252 724 B1**
(45) Date of publication and mention of the grant of the patent: **15.04.2026**
(21) Application number: 23164393.3
(22) Date of filing: 27.03.2023
(51) Int. Cl.: A61F 7/08, A61B 90/90, A61B 90/98, A61G 7/05, A47C 31/10, A47C 27/00, A61B 90/00

(54) **SYSTEM FOR COMBATING HYPOTHERMIA**
SYSTEM ZUR BEKÄMPFUNG DER HYPOTHERMIE
SYSTÈME POUR LUTTER CONTRE L'HYPOTHERMIE

(30) Priority: 31.03.2022 IT 202200006350
(43) Date of publication of application: 04.10.2023
(73) Proprietor: Epromed Srl, 25010 San Zeno Naviglio (BS) (IT)
(72) Inventor: PIZZI, Gian Vittorio, 21047 SARONNO (VA) (IT)
(74) Representative: Studio Torta S.p.A.

(56) References cited:
- WO-A1-2004/050173
- US-A1- 2004 122 419
- US-A1- 2007 083 111
- US-A1- 2011 092 890
- US-A1- 2017 135 855

## Description

### TECHNICAL FIELD

The present invention relates to the field of medical devices. In detail, the present invention relates to a system for preventing hypothermia in a patient undergoing a medical procedure, such as surgery or a diagnostic test.

### BACKGROUND

In the context of medical procedures, particularly surgical operations, it is essential to keep the temperature of the patient undergoing the medical procedure within controlled values.

Systems comprising a heating mat connected to a control unit are typically used for this purpose. The control unit regulates the heating of the mat in order to prevent the onset of hypothermia in the patient during the stages of the medical procedure, e.g. in the case of surgery: anaesthesia, the operation and waking up. US 2017/0135855 discloses an example of a system comprising a heating mat.

In order to ensure the hygiene and sterility of the mats, disposable covers are used. For this purpose, the disposable covers must be made of suitable materials - biocompatible, such as non-woven fabric or TNT -, include a double or triple layer of polypropylene to ensure fluid absorption and a layer of polyethylene to prevent liquids from reaching the surface of the mat. In addition, the covers are equipped with elastic bands at the four corners and/or a high-friction surface to ensure optimal adherence and attachment to the mat.

The use of the correct cover and its replacement with each new procedure are fundamental aspects in the preparation of an operating theatre in order to prevent the occurrence of complications during the stages of the medical procedure - for example, slippage of the cover in relation to the mat and/or contact with residual fluids from a previous medical procedure, or accidental contact of the patient's open wounds with the PVC of the heating mats or with the contaminated cover if used more than once.

In addition, US 2011/092890 discloses a sheet that manages a "microclimate". The sheet is to be placed over a person's body and includes a first layer and a flexible, fluid-proof second layer. The second layer comprises a gas-permeable region facing the person. The first layer is joined to the second layer so as to delimit a chamber. The chamber is in fluid communication with a gas nozzle, where the gas-permeable region of the second layer allows the gas to be directed towards the person's body.

WO 2004/050173 presents an electromagnetic treatment device comprising a treatment mat connected to a generation unit. The treatment mat comprises an antenna, while the generation unit comprises a start/stop button, a radio frequency generator and a verification board. In addition, there is a disposable cover that includes a label, or tag, RFID. The disposable cover system verifies that the cover is correctly arranged and that it is not previously used when the RFID tag in the cover is at a predetermined distance from the antenna of the processing mat.

US 2004/0122419 and US 2007/0083111 disclose different medical devices comprising an RFID circuit.

The Applicant therefore noted a shortage in the state of the art of solutions capable of ensuring the disposable use of a suitable cover with a corresponding specific heating mat and under the correct operating conditions.

### OBJECTS AND SUMMARY OF THE INVENTION

An object of the present invention is to overcome the drawbacks of the prior art.

The invention is defined in the appended independent claims. Embodiments of the invention are defined in the dependent claims. Methods disclosed herein may be useful for understanding the invention but do not form part of the invention.

In particular, it is the purpose of the present invention to provide a system for preventing hypothermia capable of ensuring a proper use of a disposable cover for a heating mat.

A further purpose of the present invention is to propose a system and a related kit for preventing hypothermia capable of ensuring proper use of a heating mat cover. In particular, the system and the kit are suitable to prevent the re-use of a cover for multiple medical procedures.

A further purpose of the present invention is to propose a system and a related kit for preventing hypothermia that can ensure that information about the use of the system and/or kit can be recorded and stored for long periods in a rational and effective manner.

These and other objects of the present invention are achieved by a system incorporating the characteristics of the appended claims, which form an integral part of the present description.

According to a first aspect, the present invention relates to a system for preventing hypothermia. The system comprises a heating mat comprising heating means, a control unit and a disposable cover, hereinafter referred to simply as "cover", for the heating mat.

The control unit is connected to the heating means of the heating mat to control its operation in order to bring the heating mat to a desired temperature. Advantageously, each cover is associated with a corresponding removable tag - for example, the tag is applied removably to a package of the disposable cover or to the cover itself. The tag comprises an electronic circuit adapted to transmit a radio frequency response signal in response to the reception of a radio frequency trigger signal.

In addition, the control unit comprises a receiving portion adapted to receive the removable tag associated with the cover, and is adapted to activate the heating means of the mat if it receives the response signal after transmitting the aforementioned activation signal.

The system according to the present invention is only activated when one of the disposable covers designed and supplied for the purpose of covering the heating mat is actually used to protect the mat and the removable tag is placed in the respective receiving portion. In other words, the system imposes a strict procedure of system preparation that reduces the risks of incorrect system preparation or the use of a cover unsuitable for the mat used.

In one embodiment, the system comprises a pair of mats, namely the heating mat already mentioned and a sensorised mat, preferably made of cohesive gel, comprising one or more temperature sensors. In use, the sensorised mat is placed on top of the heating mat.

Preferably, if no response signal is received within a specified time, the system is adapted to periodically transmit the activation signal until a response signal is received. Alternatively, the control unit includes means for detecting the presence of a tag in the corresponding receiving portion and, thus, transmitting the activation signal only when a tag is detected in the receiving portion. Another alternative is for the control unit to comprise user interface means and for the activation signal to be emitted only upon the input of a corresponding command by an operator.

In one embodiment, the response signal transmitted by the removable tag's electronic circuit contains a tag identification code that uniquely identifies the tag. In addition, the control unit is further adapted to activate the mat heating means if the tag code comprised in a newly received response signal is not memorised, and to memorise the tag identification code comprised in the response signal received.

Alternatively, the control unit is adapted to check whether the response signal transmitted by the electronic circuit of the removable tag comprises a unit identification code. If the response signal does not comprise a unit identification code, the control unit transmits a change signal comprising its own unit identification code stored in a memory module comprised in the control unit. Otherwise, if the response signal comprises a unit identification code, the control unit will not activate the mat heating means if the unit identification code comprised in a newly received response signal does not match its own unit identification code.

Preferably, the control unit or the electronic circuit of the tag stores an indication of a time of use of the cover. For example, the time of use originates substantially in conjunction with the recording of the tag identification code in the control unit's memory or the unit identification code in the electronic circuit of the removable tag associated with the cover. In this case, the control unit verifies that a limit use duration time has been reached on the basis of the time of use indication, and deactivates the mat heating means if said limit use duration time has been reached.

Thanks to these system configurations, it is possible to prevent the system preparation procedure from being carried out incorrectly, in particular the use of a cover that has already been used previously or that has been used for an excessive amount of time.

In one embodiment, the electronic circuit of the removable tag stores status information, for example, a status data varying between a first value indicating an unused cover and a second value indicating a used cover. In this case, the control unit is adapted to activate the mat heating means if the status information does not indicate a previous use of the cover.

Preferably, the control unit is adapted to transmit a marking signal after receiving a response signal. When the marking signal is received at the removable tag, the electronic circuit is adapted to store a status information indicating that the cover has been used. For example, the electronic circuit of the removable tag switches the status data from the first value indicating an unused cover to the second value indicating a used cover.

Thanks to this solution the possibility that a cover already used with a first mat is mistakenly used on a second mat connected to a different control unit is avoided.

In an embodiment, the control unit is adapted to determine a number of reactivations of the control unit and/or reconnections of the control unit to the heating mat based on restart information stored in the memory module comprised in the control unit and associated with a corresponding stored tag identifier or comprised in the response signal transmitted by the electronic circuit of the removable tag.

In this case, the control unit activates the mat heating means if this restart information is indicative of a number of reactivations of the control unit and/or reconnections of the control unit to the heating mat below a limit value.

Preferably, this restart indication is a restart counter. In this case, for each stored tag identification code, the control unit is adapted to store a respective restart counter, and to increment the associated restart counter each time a response signal comprising the corresponding tag identification code is received. Alternatively, the control unit is adapted to transmit an increment signal whenever it receives a response signal including its own unit identification code. In addition, the electronic circuit of the removable tag is adapted to store said restart counter, and to increment the restart counter whenever the increment signal is received.

In this way, it is possible to prevent a shutdown or temporary isolation of the control unit for safety reasons from causing a system block during a medical procedure. At the same time, limiting the number of reactivations and/or reconnections tolerated makes it possible to prevent, or at least significantly reduce, the likelihood of the same cover being mistakenly used for two or more consecutive procedures.

In an embodiment, the unique identification code contains information indicative of a model of the corresponding cover. In this case, the control unit is adapted to check whether the model of the cover indicated by the unique identification code is suitable for use with the heating mat of the system.

Checking the model of the cover in this way simply and automatically ensures that the cover dedicated to the heating mat is used.

In an embodiment of the present invention, the control unit is further configured to activate the mat heating means if the unique identification code comprised in the response signal corresponds to an expected format.

With this configuration, it is possible to more reliably ensure that the cover used is indeed suitable for the heating mat and/or that the system is not compromised. In an embodiment, the control unit is further adapted to acquire operating information while the system is in use. For example, operating information comprises, but is not limited to, a number or indication of alarms that have occurred, a minimum heating mat temperature, maximum temperature, average temperature, a set of periodically acquired heating mat temperatures, a number of control unit shutdowns, a number of heating mat disconnections, a time of use, an identification code of an activity performed in association with the use of the system, an identification code of a user of the system, at least one vital user parameter, etc.

According to the invention, the system is adapted to record temperatures exceeding a predetermined limit, an indication of an interval of time for which each of these temperatures was above the predetermined limit value and whether there was an intervention - by a system operator - to bring the temperature back below the predetermined limit value, and, possibly, an instant of time when the temperature correction intervention was performed.

The control unit transmits the acquired operating information in at least one recording signal, which is received by the electronic circuit of the removable tag. The electronic circuit of the tag is adapted to receive said recording signal, and to store the operating information contained in the recording signal.

Thanks to this solution, it is possible to collect a set of important information without the need for intervention by the user/system operator and which can be stored digitally for long periods. In particular, the removable tag can be inserted into a hospital file to store long-term data on the medical procedure in a simple and effective manner.

A different aspect of the present invention relates to a kit for a system for preventing hypothermia according to one of the preceding claims. The kit comprises at least one heating mat cover and a removable tag. The cover comprises a sheet adapted to cover at least one surface of the heating mat on which a patient is lying during use. The tag further comprises an electronic circuit adapted to transmit a radio frequency response signal in response to receiving a corresponding radio frequency trigger signal.

Preferably, the electronic circuit of the tag stores a unique identification code of the tag itself or a unique identification code of a control unit with which the tag has interacted and, preferably, at least one of a status information indicative of whether the cover has been used or not, an indication of a time of use of said cover, and an indication of a number of reactivations of the control unit and/or reconnections of the control unit to the heating mat.

In this case, the radio frequency response signal comprises said unique identification code and, preferably, at least one of said status indication, said time of use indication, and said number of reactivations/reconnections.

According to the invention, the electronic circuit of the kit's removable tag is adapted to receive a recording signal including operating information while the system is in use, and to store this operating information contained in the recording signal.

According to the invention, the stored information includes a temperature above a predetermined limit, an indication of an interval of time for which the temperature was above the predetermined limit value, an indication of a correction intervention to bring the temperature back below the predetermined limit value, and preferably, an instant of time when the correction intervention was performed.

A different aspect of the present disclosure relates to a method for controlling the operation of a system to prevent hypothermia as described above. The method envisages the control unit generating a radio frequency trigger signal, waiting for a radio frequency response signal, preferably for a predetermined time interval. If the response signal is received, preferably within this predetermined time interval, an operating state is initiated in which the control unit controls the heating means of the heating mat. Otherwise, the method envisages the control unit initiating a waiting state in which it does not control the heating means of the heating mat. Preferably, the method is repeated from the step of generating a radio frequency activation signal until the response signal is received. Alternatively, the method envisages detecting the presence of a tag in an appropriate receiving portion of the control unit or the receipt of a command from an operator before performing the above steps.

In an embodiment, the method provides that if a tag identification code, comprised in a newly received response signal, is not stored in a memory module of the control unit, the control unit activates the heating mean of the mat and stores the tag identification code comprised in the received response signal. Otherwise, the control unit preferably initiates the waiting status and reiterates the method from the step of generating a radio frequency activation signal. Alternatively, the method requires the control unit to check whether the received response signal comprises a unit identification code. If not, the control unit transmits a change signal comprising its own unit identification code stored in a memory module comprised in the control unit. This unit identification code is stored by the electronic circuit of the removable tag. If the response signal comprises a unit identification code, the control unit will not activate the mat heating means if the unit identification code comprised in a newly received response signal does not match its own unit identification code.

In one embodiment, the method envisages the control unit or tag storing an indication of a time of use of the cover. In particular, the time of use originates substantially at the same time as the recording of the tag or unit identification code. In addition, the control unit verifies that a limit use duration time has been reached based on the indication of the time of use, and deactivates the mat heating means if said limit use duration time has been reached.

In one embodiment, the method comprises the control unit checking whether status information comprised in the response signal indicates a previous use of the cover and, if so, not activating the mat heating means. Otherwise, if the status information does not indicate a previous use of the cover, the control unit activates the mat heating means and transmits a marking message. Preferably, upon receipt of the marking message, the method requires the removable tag to change the status information to indicate the use of the cover associated with the removable tag.

In one embodiment, for each stored unique identification code, the method comprises the control unit or tag storing the restart information - mentioned above - associated with each unique identification code, and activating the mat heating means if it is verified that the number of reactivations of the control unit and/or reconnections of the control unit to the heating mat is less than a limit value based on said restart information associated with the corresponding unique identifier.

In one embodiment, the method envisages the control unit verifying that the unique identification code is of an expected format and/or the unique identification code being associated with an expected model of cover suitable for use with the heating mat connected to the control unit.

The method in its various embodiments described above allows obtaining corresponding advantages to what is disclosed above for the system in its various embodiments.

Further features and advantages of the present invention will be more evident from the description of the accompanying drawings.

### BRIEF DESCRIPTION OF THE DRAWINGS

The invention is described hereinbelow with reference to certain examples provided by way of non-limiting example and illustrated in the accompanying drawings. These drawings illustrate different aspects and embodiments of the present invention and reference numerals illustrating structures, components, materials and/or similar elements in different drawings are indicated by similar reference numerals, where appropriate.
Figure 1 is a schematic view of a system for preventing hypothermia in a patient according to an embodiment of the present invention;
Figure 2 is a schematic view of a system for preventing hypothermia in a patient of Figure 1, in a use configuration;
Figure 3 is a block diagram of the control unit of the system for preventing hypothermia in a patient of Figure 1;
Figure 4 is a flowchart of a method for preparing the system of Figure 1;
Figure 5 is a flow diagram of a method for verifying the cover for a heating mat according to an embodiment of the present invention;
Figure 6 is a flow diagram of a method for verifying the cover for a heating mat according to an alternative embodiment of the present invention, and
Figure 7 is a flow diagram of a method for recording information on a tag associated with a cover for a heating mat, according to an embodiment of the present invention.

### DETAILED DESCRIPTION OF THE INVENTION

While the invention is susceptible to various modifications and alternative constructions, certain preferred embodiments are shown in the drawings and are described hereinbelow in detail. It must in any case be understood that there is no intention to limit the invention to the specific embodiment illustrated, but, on the contrary, the invention intends covering all the modifications, alternative and equivalent constructions that fall within the scope of the invention as defined in the claims.

The use of "for example", "etc.", "or" indicates non-exclusive alternatives without limitation, unless otherwise indicated. The use of "includes" means "includes, but not limited to" unless otherwise indicated.

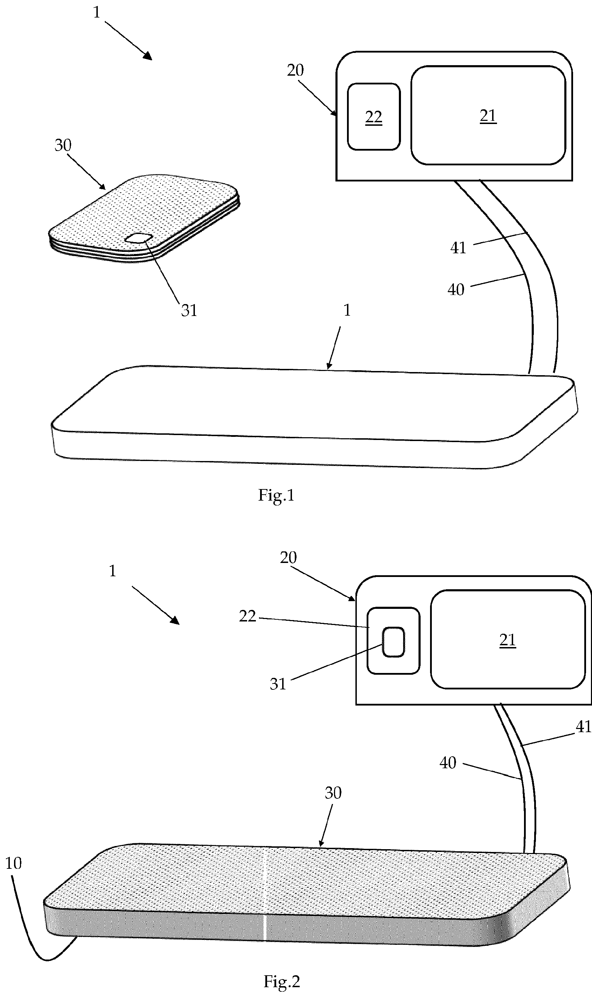

With reference to Figure 1 and Figure 2, a system 1 for preventing hypothermia in a patient according to an embodiment of the present invention comprises a heating mat 10, a control unit 20 and a disposable cover, simply cover 30 in the following, for the heating mat 10. The cover 30 is shown in a folded configuration in Figure 1 and, in a use configuration, applied to heating mat 10 in Figure 2.

The heating mat 10, in a known way, comprises electrical heating means (not illustrated here for simplicity's sake) and, optionally, one or more temperature sensors (not illustrated), which may be removable.

The heating mat 10 is connected to the control unit 20 by means of appropriate wiring 40. In particular, the heating means are connected to the wiring in such a way that they receive from the control unit 20 the electrical energy required to power the heating means to reach a desired heating temperature. In addition, any one or more sensors are connected to the control unit 20 via respective dedicated wiring 41 in order to transmit the temperature measurements taken to the control unit 20.

In addition, the control unit 20 comprises a user interface 21 adapted to receive instructions and provide information to a user (not illustrated, typically a doctor or nurse). In a non-limiting way, the user interface 21 comprises one or more buttons, knobs and/or levers to receive instructions and a screen to provide information to the user, or a touch screen with input and output functions. In the embodiments, of the present invention, the control unit 20 also comprises a receiving portion 22, adapted to receive, i.e. to house (as illustrated in Figure 2), a tag 31 associated with the cover 30, which is described below.

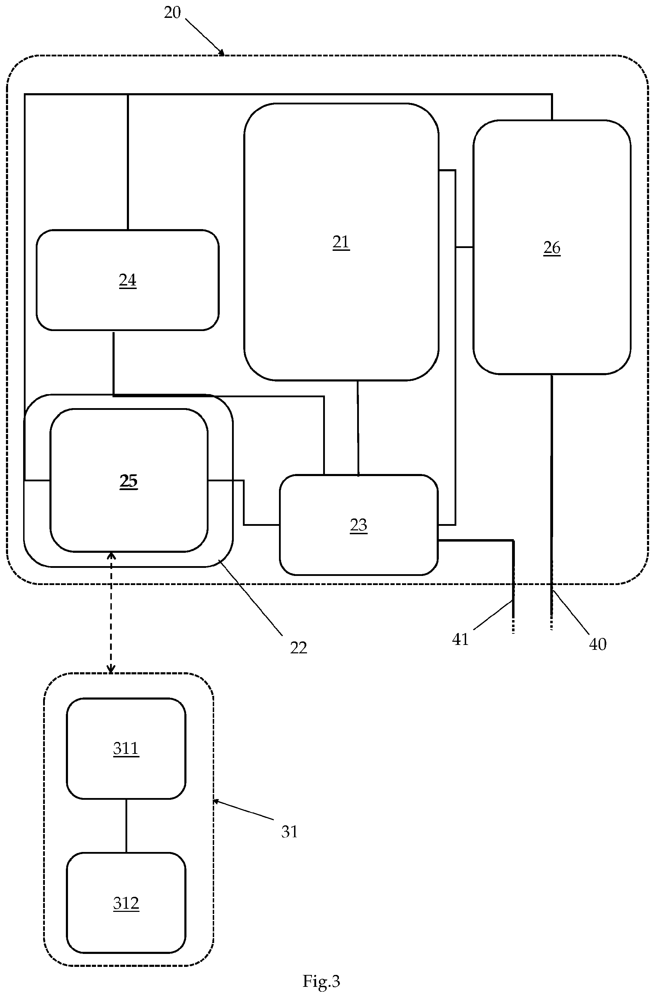

As illustrated in the block diagram of Figure 3, the control unit 20 comprises a processor module 23, a memory module 24, a transceiver module 25 and a power supply module 26. Preferably, the transceiver module 25 is positioned at the receiving portion 22.

In short, the processor module 23 of the control unit 20 is connected to the other modules 24-26 and to the user interface 21 to control their operation - for example, according to instructions or software or firmware stored in the memory module 24. Similarly, the power supply module is connected to the other modules 23-25 and to the user interface 21 and to the heating means of the heating mat 10 - via the wiring 40 - to supply power thereto.

The cover 30 is a substantially rectangular sheet of such dimensions as to cover the heating mat 10. In particular, the cover 30 is of such a size and shape that it completely covers at least the upper surface of the heating mat 10, i.e. the portion of the heating mat 10 on which a patient is lying during a medical procedure.

Preferably, the cover 30 comprises an elastic element (not illustrated) at a perimeter edge or at the four corners of the rectangle in order to ensure precise and reliable application of the cover 30 on the heating mat 10. In addition or as an alternative, the cover 30 comprises a high-friction surface - e.g. rubberized - suitable for contact with the heating mat 10, so as to ensure optimal adherence and attachment of the cover 30 to the heating mat 10.

Advantageously, the cover 30 is made of a waterproof and tear-resistant material, possibly also sterile. For example, the cover 30 is made of a non-woven fabric or TNT. Preferably, the cover 30 comprises a double or triple layer of TNT and a layer of polyethylene to ensure optimal isolation of the heating mat 10 from the patient and/or fluids dispersed during the medical procedure to which the patient is subjected.

As mentioned above, in the embodiments of the present invention, the cover 30 comprises a removable tag 31. For example, the tag is an essentially disc-shaped flexible foil with a diameter of approximately 25 mm with a self-adhesive layer. The removable tag 31 comprises an electronic circuit suitable for exchanging data with the control unit 20, as described below. Preferably, the tag 31 comprises a Radio-Frequency IDentification (RFID) electronic circuit. In more detail, the electronic circuit of the tag 31 comprises an antenna 311 and a controller module 312 connected together, as shown in the block diagram of Figure 3.

For example, the tag is adapted to exchange signals at a frequency of around 13.56 MHz and has a memory of at least 1 Kbyte. Each label has a unique serial number.

Prior to performing a medical procedure, an operator (not shown) performs a method 1000 of preparing the system 1 by applying the cover 30 to the heating mat 10 so as to adequately cover it (step 1001). In addition, the tag 31 is removed from the cover 30 and placed on the receiving portion 22 (step 1003, as shown in Figure 2). Finally, the control unit 20 is started (step 1005)

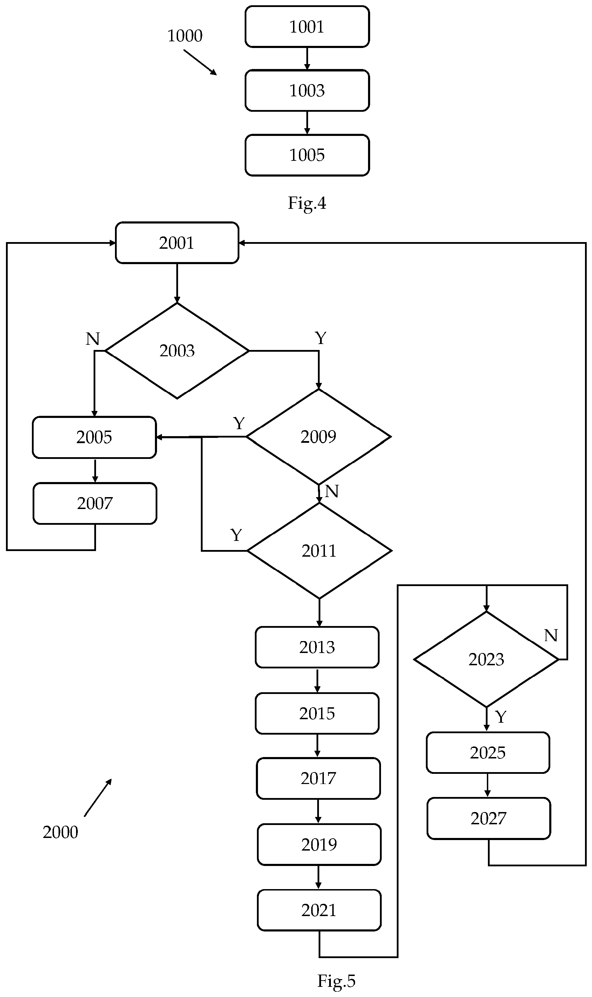

Once switched on, the control unit 20 of the system 1 is adapted to perform a method 2000 for verifying the heating mat cover according to an embodiment of the present disclosure, of which Figure 4 is a flow chart.

The control unit 20 generates an SA activation signal via the transceiver module 25 (step 2001) and waits for an SR response signal for a predetermined time interval (decision step 2003). The SA activation signal is a radio frequency signal, in the example considered with a frequency comprised within the bands used by RFID devices.

If a tag 31 is not placed in the receiving portion 22, no SR response signal is received within the predetermined time interval (output branch N of step 2003), the control unit 20 enters a waiting state in which it does not supply power to the heating means of the heating mat 10 (step 2005). Preferably, an error message is presented via the user interface 21 (step 2007). For example, the error message instructs the operator to apply the cover 30 to the heating mat 10 and place the corresponding tag 31 in the receiving portion 22. Subsequently, the control unit sends the trigger signal SA again, i.e. the method resumes from step 2001.

Otherwise, if the tag 31 is placed in the receiving portion 22, the latter generates the SR response signal. Advantageously, the SR response signal transmitted by the tag 31 includes a CID identification code stored in the controller module 312, which uniquely identifies the cover 30. Preferably, the SR response signal also includes status information S, indicative of a previous use of the cover 30. For example, status information S is data stored in a memory area (not shown) of the controller module 312 of the tag 31. This can take a first value - indicative of the fact that the cover 30 has never been used - or a second value - indicative of the fact that the cover 30 has been used.

Accordingly, the control unit 20 receives the SR response signal within the predetermined time interval (output branch Y of step 2003) and checks whether the CID code contained in the SR response signal is recorded in its memory module 24 (decision step 2009).

In the event that the CID code contained in the response signal SR is present in the memory module 24 (output branch Y of step 2009) the control unit 20 enters the waiting state in which it does not supply power to the heating means of the heating mat 10 and, preferably, the error message is presented via the interface of the control unit 20. In other words, the method 2000 continues to steps 2005 and 2007 described above. In this case, the error message instructs the operator to apply a new cover 30 to the heating mat 10 and place the corresponding tag 31 in the receiving portion 22. Subsequently, the control unit sends the trigger signal SA again, i.e. the method resumes from step 2001.

In the event that the CID code contained in the response signal SR is not present in the memory module 24 (output branch N of step 2009), the control unit 20 checks whether the status information S indicates a previous use of the cover 30 (decision step 2011).

In the event that the status information S indicates a previous use of the cover 30 (output branch Y of step 2011), the control unit 20 enters the waiting state in which it does not supply power to the heating means of the heating mat 10 and, preferably, the error message is presented via the interface of the control unit 20. In other words, the method 2000 continues to steps 2005 and 2007 described above. In this case, the error message instructs the operator to apply a new cover 30 to the heating mat 10 and place the corresponding tag 31 in the receiving portion 22. Subsequently, the control unit sends the trigger signal SA again, i.e. the method resumes from step 2001.

Alternatively, if the status information S does not indicate a previous use of the cover 30 (output branch N of step 2011), the control unit 20 stores the CID code in the memory module 24 (step 2013), stores a time marking, or timestamp ts, of activation of the cover 30 (step 2015), and transmits a marking signal SM, which is received by the tag 31 that switches the stored status information S from the first value to the second value (step 2017).

Thereafter, the control unit 20 enters an operational state in which it allows the supply of electricity to the heating means of the heating mat 10 (step 2019) automatically and/or according to commands entered by the operator (step 2021).

In addition, the control unit 20 verifies whether a time elapsed since the start of use of the cover 30, indicated by the timestamp ts, is equal to - i.e., has reached - a use time limit Tth (decision step 2023). The use time limit Tth is predetermined and stored in the memory module 24 of the control unit 20. For example, the use time limit Tth is comprised between 1 hour and 20 hours, such as 10 or 15 hours.

In the event that the lapsed time from the start of the use of the cover 30 is not greater than or equal to the use time limit Tth (output branch N of step 2023), the control unit 20 continues to monitor the use clock CK to verify that the use time limit Tth has been reached (i.e. the operation returns to step 2023). When, on the other hand, the elapsed time from the start of use of the cover 30 is greater than or equal to the use time limit Tth (output branch Y of step 2023), the control unit 20 generates a warning via the user interface 21 to inform the user that the cover 30 is to be replaced due to having reached an extended use limit (step 2025) and goes into the waiting state in which it does not supply power to the heating means of the heating mat 10 (step 2027 analogous to step 2005). Optionally, the waiting state is activated after a tolerance time interval after the generation of the warning in step 2025. Subsequently, the control unit sends the trigger signal SA again, i.e. the method resumes from step 2001.

The Applicant has noted that during a medical procedure, in particular during surgical operations, it may be necessary to deactivate the heating mat 10. For example, if a defibrillator is used on the patient, the heating mat 10 is electrically isolated from the control unit 20, or the control unit 20 is switched off, in order to avoid damage thereto due to the electrical discharge imposed by the defibrillator.

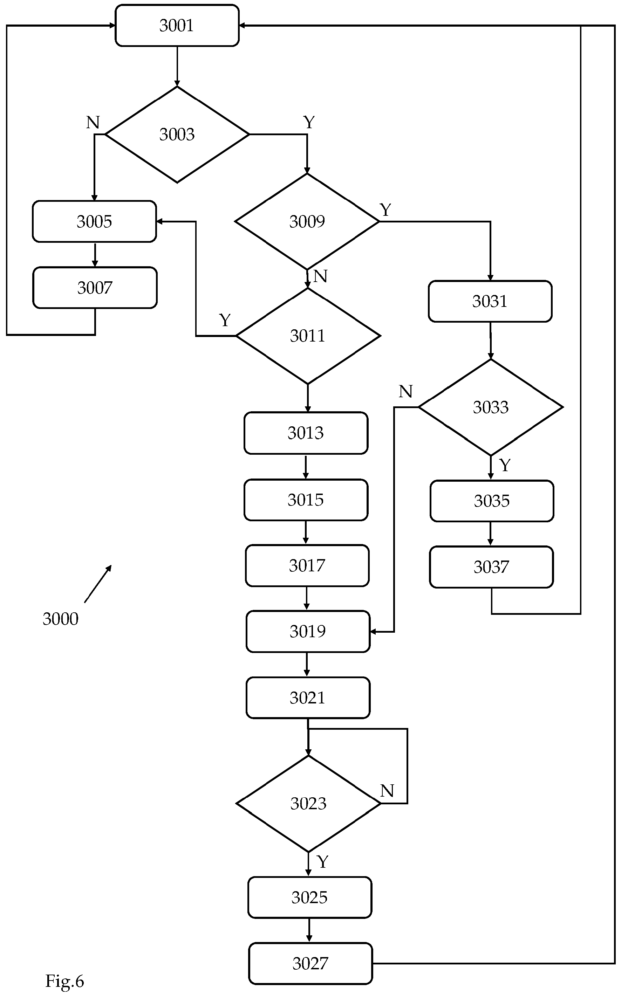

Accordingly, the Applicant has developed an alternative verification method 3000 - of which Figure 6 is a flowchart - which makes it possible to identify a temporary interruption in the operation of system 1 that is envisaged or necessary for the success of the medical operation and to ensure continued use of the system 1.

The method 3000 is iterated each time the control unit 20 is switched on and each time the control unit is reconnected to the heating mat 10.

The method 3000 comprises steps 3001 - 3027 corresponding to steps 2001 - 2027 described above and not repeated here for brevity. In addition, the method 3000 comprises the following additional steps.

When the control unit verifies that the CID code contained in the response signal SR is present in the memory module 24 (output branch Y of step 3009), the control unit 20 increments a restart counter CR (step 3031) and verifies whether the restart counter CR is equal to - i.e., has reached - a limit number of restarts Rth (decision step 3033).

When the restart counter CR is equal to the limit number of restarts Rth (output branch Y of step 3033), the control unit 20 generates a warning via the user interface 21 to inform the user that the cover 30 is to be replaced due to having reached an extended use limit (step 3035) and goes into the waiting state where it does not supply power to the heating means of the heating mat 10 (step 3037 analogous to step 3005). Optionally, the waiting state is started after a tolerance time interval after the generation of the warning in step 3035. Subsequently, the control unit sends the trigger signal SA again, i.e. the method resumes from step 3001.

If, on the other hand, the restart counter CR is not equal to the limit number of restarts Rth (output branch N of step 3033), the control unit 20 enters the operational state (i.e. operation continues to step 3019).

However, it is clear that the above examples must not be interpreted in a limiting sense and the invention thus conceived is susceptible of numerous modifications and variations.

For example, although the removable tag preferably comprises an RFID-type circuit, there is nothing to prevent other embodiments (not illustrated) in which one or more other communication technologies such as NFC, Zigbee, Bluetooth, etc. are used.

In addition, in one embodiment, one or more covers each with a related tag are supplied as a spare kit, preferably individually packaged, optionally in sterile packaging. In this case, the electronic circuit of each tag is adapted to generate a response signal comprising a respective unique identification code, different from the others.

As will be clear to a person skilled in the art, one or more of the steps of the methods described above may be performed in parallel with each other or in a different order from the one presented above. In particular, steps 2023-2027 of the method 2000 and steps 3023-3027 of the method 3000, i.e. the steps associated with the verification of the attainment of the use time limit, may be performed in parallel with the steps following the recording of the timestamp (step 2015 or 3015). Similarly, one or more optional steps can be added or removed from one or more of the procedures described above.

For example, in a simplified embodiment (not illustrated), the control unit is adapted to enter the operational state simply upon receipt of the response signal, without performing a verification of the tag identification code and/or status signal.

In one embodiment, the heating mat can be electrically isolated from the control unit without the latter being deactivated. In this case, the method 3000 described above is repeated each time the electrical connection between the control unit and the heating mat is re-established, as well as each time the control unit is reactivated.

In one embodiment (not shown), the system comprises a pair of mats: the heating mat described above and a sensorised mat. Specifically, the heating mat comprises heating means, while the sensorised mat comprises temperature sensors. In use, the sensorised mat is placed on top of the heating mat. Advantageously, the sensorised mat is a mat with an anti-bedsore function. In this case, the cover is suitable to cover both the mats stacked or, at least, the mat on top - i.e. the sensorised mat in this case.

In an alternative embodiment, one or more sensors configured to detect the presence of a tag are positioned at the receiving portion. For example, the control unit includes an optical sensor that detects the presence of a tag positioned in the receiving portion. In this case, the activation signal is transmitted by the control unit only after a tag has been detected in the receiving portion.

In an alternative embodiment, the operator enters confirmation of the positioning of the cover on the heating mat and/or the tag in the receiving portion via the user interface. For example, the activation signal is transmitted by the control unit after a corresponding activation command provided by the user is received - indicative of the fact that the tag has been placed in the receiving portion and the cover covers the heating mat.

In one embodiment, the reactivation counter is used as status information. In this case, a count of zero indicates that the cover has never been used, while a non-zero count indicates that the cover has been used.

In an alternative embodiment, the timestamp is stored in the memory area of the electronic tag circuit rather than in the control unit.

In this case, after verifying the identification code and the status signal, the verification method requires the control unit to check whether the tag stores a timestamp. If there is no timestamp, the control unit sends a start signal comprising the current timestamp to be stored. On the contrary, if a timestamp is present, the control unit checks whether the time limit has elapsed, in a similar way to what has been described above.

In addition or alternatively, the restart counter is stored in the memory area of the electronic circuit board of the tag.

In this case, after having verified the identification code, the status signal and the time of use, the verification method requires the control unit to check whether the restart counter stored on the tag is higher than the number of limit starts in a similar way to what has been described above. In addition, the control unit transmits an appropriate increment signal every time the restart counter needs to be incremented.

In an alternative embodiment, an identification code associated with the control unit is used, rather than the tag.

In this case, the status information indicates that the cover has never been used or indicates the identification code of the control unit connected to the heated mat on which the cover is applied. For example, the status information has a null value when the cover has not been used, while it stores an identification code of the control unit with which it is used, e.g. a unique alphanumeric string for each control unit.

In more detail, the response signal sent by the tag comprises status information. The control unit verifies the content of the status information. If the status information does not show a previous use, the control unit transmits a status change signal containing the control unit's identification code, which is stored by the electronic circuit of the tag and is brought into the operational status. Otherwise, if the status information contains an identification code, the control unit will only enter the operational state if the identification code corresponds to its own control unit code, and - similarly to what has been described above - if the time of use and, preferably, the number of reactivations/reconnections are below the respective limit values.

In an alternative embodiment, the control unit is adapted to verify a validity of each received identification code. Preferably, the control unit is adapted to verify whether each received identification code corresponds to an expected format. For example, the processor module comprises a verification algorithm adapted to provide a positive result if the unique identification code matches the expected format or otherwise a negative result. In this case, the control unit only goes into the operational state if the check gives a positive result.

In addition or alternatively, the unique identification code contains information indicative of a model of the corresponding cover. In this case, the control unit is adapted to check whether the model of the cover indicated by the unique identification code is suitable for use with the heating mat of the system and to activate the heating means only if this is the case.

In one embodiment, the control unit associates a time counter with the newly stored identification code instead of a timestamp. In this case, the counter is incremented with each pulse generated by an internal clock in the control unit, and it is checked whether the counter has reached a limit number corresponding to the permitted use of the mat. More generally, the control unit stores an indication of the time of use of each cover and checks whether a limit, i.e. maximum, time of use has been reached.

In addition, there is nothing to prevent the control unit from storing several timestamps associated with the same identification code - for example, in a vector - and using the number of timestamps stored, or using the timestamps to calculate the total time of use and/or a total time of inactivity as a criterion for determining whether to enter the operational state or the waiting state and/or a number of restarts in order to assess a limit of use of the cover.

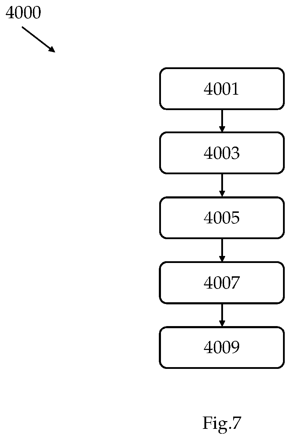

In one embodiment, during the procedure and/or at the end of the procedure the control unit is adapted to perform a recording method 4000 - of which Figure 7 is a flow chart.

The method 4000 requires the control unit to acquire operating information of the system 1 during the execution of a medical procedure (step 4001). In a non-limiting manner, the information acquired comprises:
- a number or indication of alarms that have occurred (generated by the control unit 20),
- a minimum heating mat temperature,
- a maximum temperature,
- an average temperature,
- a set of heating mat temperatures acquired periodically,
- a number of control unit shutdowns 20,
- a number of heating mat disconnections 10,
- a time of use,
- an identification code for the operation carried out,
- an identification code of the assisted patient,
- one or more vital signs of the assisted patient (e.g. body temperature, heartbeat, saturation provided by one or more apparatuses external to the system 1), etc.

The control unit 20 transmits the acquired operating information in at least one recording message (step 4003). For example, a single recording message is transmitted at the end of the medical procedure, or a plurality of recording messages are transmitted at respective times during and/or at the end of the medical procedure.

The removable tag 31, in particular the electronic circuit receives the one or more recording messages (step 4005) and stores the operating information in the memory area of the controller module 312 of the tag 31 (step 4007).

Subsequently, the tag 30 can be applied to the patient's medical record (not shown) for any subsequent analysis of the operation of the system 1 during the medical procedure (step 4009).

For example, in an embodiment, it is envisaged that when a temperature above a predetermined limit value is detected - by means of the temperature sensors arranged in the mat 10 or in the sensorised mat - the detected temperature is acquired by the control unit together with an indication of an interval of time for which the temperature remained above the predetermined limit, whether there was an intervention by an operator of the system 1 to bring the temperature back below the predetermined limit and, possibly, an instant of time in which the temperature correction intervention was performed. This information is entered into the recording message and transmitted by the control unit, so that it is received and recorded by the tag 30 (i.e., stored in the memory area of the controller module 312).

Subsequently, the tag 30 can be interrogated via an appropriate reading device to read the information stored in the tag 30. In other words, the tag 30 has the additional function of storing information that can describe, i.e. reconstruct, the course of a medical activity in which the system 1 was used. In conclusion, the tag 30 acts as a kind of "black box" ensuring the storage of previously unavailable information on a medical activity performed.

Naturally, all the details can be replaced with other technically-equivalent elements.

In conclusion, the materials used, as well as the contingent shapes and dimensions of the aforementioned devices, apparatuses and terminals, may be any according to the specific implementation requirements without thereby abandoning the scope of protection of the following claims.

## Claims

1. System (1) to prevent hypothermia comprising:
a heating mat (10) comprising heating means and at least one temperature sensor,
a control unit (20), and
a disposable cover (30) for the heating mat (10),
where the control unit (20) is connected to the heating means and the at least one temperature sensor of the heating mat (10) to control its operation in order to bring the heating mat (10) to a desired temperature,
wherein
it further comprises a removable tag (31) associated with the cover (30), said removable tag (31) comprising an electronic circuit (311, 312) adapted to transmit a radio frequency response signal in response to the reception of a radio frequency trigger signal, and
in that
the control unit (20) comprises a receiving portion (22) adapted to receive the removable tag (31) once separated from the cover (30), and
in that
the control unit is adapted to:
not activate the mat heating means (10) if it does not receive the response signal after transmitting the activation signal,
activate the mat heating means (10) if it does receive the response signal after transmitting the activation signal,
when a temperature above a predetermined limit value is detected, acquire (4001) operating information during the use of the system (1), the operating information comprising:
- the temperature above the predetermined limit value,
- an indication of a time interval for which the temperature remained above the predetermined limit value,
- an indication of a correction intervention to bring the temperature below the predetermined limit value, and
- preferably, an instant of time when the correction was performed, and transmit the operating information acquired in at least one recording signal (4003), and
wherein the electronic circuit (311, 312) of the removable tag (31) is adapted to:
receive said recording signal, and
store the operating information contained in the recording signal.

2. System (1) according to claim 1, wherein the response signal transmitted by the electronic circuit (311, 312) of the removable tag (31) contains a tag identification code, and
wherein the control unit (20) is further adapted to:
activate the mat heating means (10) if the tag identification code comprised in a newly received response signal is not stored in a memory module (24) comprised in the control unit, and
store the tag identification code comprised in the response signal received in the memory module (24).

3. The system (1) according to claim 1, wherein the control unit (20) is further configured to:
check whether the response signal transmitted by the electronic circuit (311, 312) of the removable tag (31) comprises a unit identification code,
if the response signal does not comprise a unit identification code, transmit a change signal comprising its own unit identification code stored in a memory module (24) comprised in the control unit (20), and
not activate the heating means of the mat (10) if the unit identification code comprised in a newly received response signal does not match your own unit identification code.

4. System (1) according to claim 2 or 3, wherein the control unit (20) is further adapted to:
determine a time of use of the cover (30) on the basis of time of use information stored in the memory module (24) comprised in the control unit (20) or comprised in the response signal transmitted by the electronic circuit (311, 312) of the removable tag (31),
verify the achievement of a limit use duration time based on the time of use indication, and
deactivate the heating means of the mat (10) if it is verified that said limit use duration has been reached.

5. System (1) according to claim 4, wherein the control unit (20) is further adapted to:
store the time of use information from an instant of time in which said identification code is stored, or
transmit a use start signal comprising time of use information, and
wherein the electronic circuit (311, 312) of the removable tag (31) is adapted to:
receive the use start signal, and
store the time of use information comprised in said use start signal.

6. System (1) according to any one of the preceding claims, wherein the response signal transmitted by the electronic circuit (311, 312) of the removable tag (31) contains status information,
wherein the control unit is adapted to activate the heating means of the mat (10) if the status information does not indicate a previous use of the cover (30), and to transmit a marking signal after receiving a response signal, and
wherein the electronic circuit (311, 312) of the removable tag (31) is adapted to:
receive said marking signal, and
store status information indicative of a use of the cover (30).

7. System (1) according to any one of the preceding claims dependent on claim 2 or claim 3, wherein the control unit (20) is further adapted to:
determine a number of reactivations of the control unit (20) and/ or reconnections of the control unit (20) to the heating mat (10) based on restart information stored in the memory module (24) comprised in the control unit (20) and associated with a corresponding stored tag identifier or comprised in the response signal transmitted by the electronic circuit (311, 312) of the removable tag (31), and
activate the mat (10) heating means if this restart information is indicative of a number of reactivations of the control unit (20) and/or reconnections of the control unit (20) to the heating mat (10) below a limit value.

8. System (1) according to claim 7, wherein said restart indication is a restart counter, and
wherein, for each stored tag identification code, the control unit is further adapted to:
store a respective restart counter, and
increment the associated restart counter each time a response signal comprising the corresponding tag identification code is received, or
wherein the control unit is adapted to:
transmit an increment signal, each time it receives a response signal comprising its unit identification code, and
wherein the electronic circuit (311, 312) of the removable tag (31) is adapted to:
store said restart counter, and
increment the restart counter each time the increment signal is received.

9. System (1) according to any one of the preceding claims, wherein said operating information comprises one or more among:
- a number or indication of alarms that have occurred (generated by the control unit 20),
- a minimum heating mat temperature,
- a maximum temperature,
- an average temperature,
- a set of heating mat temperatures acquired periodically,
- a number of control unit shutdowns 20,
- a number of heating mat disconnections 10,
- a time of use,
- an identification code for the operation carried out,
- an identification code of the assisted patient, and
- at least one vital sign of the assisted patient.

10. Kit (30,31) for a system (1) to prevent hypothermia comprising a heating mat (10) provided with heating means, and a control unit (20) adapted to control the operation of the heating mat, the kit comprising:
at least one cover (30) for the heating mat (10) and a removable tag (31), said cover (30) comprising a sheet suitable for covering at least one support surface of the heating mat (10) on which a patient is lying during use, and wherein
the removable tag (31) comprises an electronic circuit (311, 312) adapted to:
transmit a radio frequency response signal in response to the reception of a corresponding radio frequency trigger signal,
receive a recording signal including operating information during the use of the system (1), and
store the operating information contained in the recording signal, the operating information comprising:
- a temperature above a predetermined limit value,
- an indication of a time interval for which the temperature remained above the predetermined limit value,
- an indication of a correction intervention to bring the temperature below the predetermined limit value, and
- preferably, an instant of time when the correction was performed.

11. Kit (30,31) according to claim 10, wherein electronic circuit (311, 312) is adapted to store a unique tag or unit identification code and, preferably, at least one among: status information indicative of whether or not the cover (30) has been used, an indication of a time of use of said cover (30), and an indication of a number of reactivations of the control unit (20) and/or reconnections of the control unit (20) to the heating mat (10), and
wherein the radio frequency response signal comprises said unique identification code and, preferably, at least one of said status indication, said use time indication, and said number of reactivations/reconnections.

## Patentansprüche

1. System (1) zur Unterkühlungsverhinderung, umfassend:
eine Heizmatte (10) mit Heizmitteln und mindestens einem Temperatursensor,
eine Steuereinheit (20) und
eine Einwegabdeckung (30) für die Heizmatte (10),
wobei die Steuereinheit (20) mit den Heizmitteln und dem mindestens einen Temperatursensor der Heizmatte (10) verbunden ist, um deren Betrieb zu steuern, um die Heizmatte (10) auf eine gewünschte Temperatur zu bringen,
wobei
es ferner ein abnehmbares Tag (31) umfasst, das mit der Abdeckung (30) assoziiert ist, wobei das abnehmbare Tag (31) eine elektronische Schaltung (311, 312) umfasst, die angepasst ist, um ein Hochfrequenzantwortsignal als Reaktion auf den Empfang eines Hochfrequenztriggersignals zu übermitteln, und
dadurch, dass
die Steuereinheit (20) einen Aufnahmeabschnitt (22) umfasst, der angepasst ist, das abnehmbare Tag (31) aufzunehmen, sobald es von der Abdeckung (30) getrennt ist, und
dadurch, dass
die Steuereinheit angepasst ist, um:
die Mattenheizmittel (10) nicht zu aktivieren, wenn sie nach der Übermittlung des Aktivierungssignals das Antwortsignal nicht empfängt,
die Mattenheizmittel (10) zu aktivieren, wenn sie das Antwortsignal nach der Übermittlung des Aktivierungssignals empfängt,
wenn eine Temperatur über einem vorgegebenen Grenzwert detektiert wird, Betriebsinformationen während der Nutzung des Systems (1) (4001) zu erfassen, wobei die Betriebsinformationen Folgendes umfassen:
- die Temperatur über dem vorgegebenen Grenzwert,
- eine Anzeige eines Zeitintervalls, in dem die Temperatur über dem vorgegebenen Grenzwert blieb,
- eine Anzeige eines Korrektureingriffs, um die Temperatur unter den vorgegebenen Grenzwert zu bringen, und
- vorzugsweise einen Zeitpunkt, zu dem die Korrektur durchgeführt wurde, und die in mindestens einem Aufzeichnungssignal (4003) erfassten Betriebsinformationen zu übermitteln, und
wobei die elektronische Schaltung (311, 312) des abnehmbaren Tags (31) dazu angepasst ist:
das Aufzeichnungssignal zu empfangen, und
die im Aufzeichnungssignal enthaltenen Betriebsinformationen zu speichern.

2. System (1) nach Anspruch 1, wobei das von der elektronischen Schaltung (311, 312) des abnehmbaren Tags (31) übermittelte Antwortsignal einen Tag-Identifikationscode enthält und wobei die Steuereinheit (20) ferner dazu angepasst ist:
die Mattenheizmittel (10) zu aktivieren, wenn der in einem neu empfangenen Antwortsignal enthaltene Tag-Identifikationscode nicht in einem in der Steuereinheit enthaltenen Speichermodul (24) gespeichert ist, und
den Tag-Identifikationscode zu speichern, der in dem im Speichermodul (24) empfangenen Antwortsignal enthalten ist.

3. System (1) nach Anspruch 1, wobei die Steuereinheit (20) ferner dazu konfiguriert ist:
zu überprüfen, ob das von der elektronischen Schaltung (311, 312) des abnehmbaren Tags (31) übermittelte Antwortsignal einen Einheitsidentifikationscode umfasst,
wenn das Antwortsignal keinen Einheitsidentifikationscode enthält, ein Änderungssignal zu übermitteln, das seinen eigenen Einheitsidentifikationscode umfasst, der in einem in der Steuereinheit (20) enthaltenen Speichermodul (24) gespeichert ist, und
die Heizmittel der Matte (10) nicht zu aktivieren, wenn der in einem neu empfangenen Antwortsignal enthaltene Einheitsidentifikationscode nicht mit seinem eigenen Einheitsidentifikationscode übereinstimmt.

4. System (1) nach Anspruch 2 oder 3, wobei die Steuereinheit (20) ferner dazu angepasst ist:
eine Nutzungszeit der Abdeckung (30) auf der Grundlage von Nutzungszeitinformationen zu bestimmen, die in dem Speichermodul (24) gespeichert sind, das in der Steuereinheit (20) enthalten ist oder in dem Antwortsignal enthalten sind, das von der elektronischen Schaltung (311, 312) des abnehmbaren Tags (31) übermittelt wird,
das Erreichen einer Grenznutzungsdauerzeit anhand der Nutzungszeitanzeige zu überprüfen, und
die Heizmittel der Matte (10) zu deaktivieren, wenn überprüft wird, dass die Grenznutzungsdauer erreicht wurde.

5. System (1) nach Anspruch 4, wobei die Steuereinheit (20) ferner dazu angepasst ist:
die Nutzungszeitinformationen ab einem Zeitpunkt zu speichern, zu dem der Identifikationscode gespeichert wird, oder
ein Nutzungsstartsignal mit Nutzungszeitinformationen zu übermitteln, und
wobei die elektronische Schaltung (311, 312) des abnehmbaren Tags (31) angepasst ist, um: das Nutzungsstartsignal zu empfangen, und
die in dem Nutzungsstartsignal enthaltene Nutzungszeitinformation zu speichern.

6. System (1) nach einem der vorhergehenden Ansprüche, wobei das von der elektronischen Schaltung (311, 312) des abnehmbaren Tags (31) übermittelte Antwortsignal Statusinformationen enthält,
wobei die Steuereinheit so angepasst ist, dass sie die Heizmittel der Matte (10) aktiviert, wenn die Statusinformationen keine vorherige Nutzung der Abdeckung (30) anzeigen, und dass sie nach dem Empfang eines Antwortsignals ein Markierungssignal übermittelt, und
wobei die elektronische Schaltung (311, 312) des abnehmbaren Tags (31) dazu angepasst ist: das Markierungssignal zu empfangen, und
Statusinformationen zu speichern, die eine Nutzung der Abdeckung (30) anzeigen.

7. System (1) nach einem der vorangehenden Ansprüche, abhängig von Anspruch 2 oder Anspruch 3, wobei die Steuereinheit (20) ferner dazu angepasst ist:
eine Anzahl von Reaktivierungen der Steuereinheit (20) und/oder Wiederverbindungen der Steuereinheit (20) mit der Heizmatte (10) auf der Grundlage von Neustartinformationen zu bestimmen, die in dem Speichermodul (24) gespeichert sind, das in der Steuereinheit (20) enthalten ist und mit einem entsprechenden gespeicherten Tag-Identifizierer assoziiert ist oder in dem Antwortsignal enthalten sind, das von der elektronischen Schaltung (311, 312) des abnehmbaren Tags (31) übermittelt wird, und
die Heizmittel der Matte (10) zu aktivieren, wenn diese Neustartinformationen eine Anzahl von Reaktivierungen der Steuereinheit (20) und/oder Wiederverbindungen der Steuereinheit (20) mit der Heizmatte (10) unterhalb eines Grenzwerts anzeigen.

8. System (1) nach Anspruch 7, wobei die Neustartanzeige ein Neustartzähler ist, und
wobei für jeden gespeicherten Tag-Identifikationscode die Steuereinheit ferner dazu angepasst ist:
einen jeweiligen Neustartzähler zu speichern, und
den assoziierten Neustartzähler jedes Mal zu erhöhen, wenn ein Antwortsignal mit dem entsprechenden Tag-Identifikationscode empfangen wird, oder
wobei die Steuereinheit dazu angepasst ist:
jedes Mal, wenn sie ein Antwortsignal mit ihrem Einheitsidentifikationscode empfängt, ein Erhöhungssignal zu übermitteln und
wobei die elektronische Schaltung (311, 312) des abnehmbaren Tags (31) angepasst ist, um: den Neustartzähler zu speichern, und
den Neustartzähler jedes Mal zu erhöhen, wenn das Erhöhungssignal empfangen wird.

9. System (1) nach einem der vorhergehenden Ansprüche, wobei die Betriebsinformationen eine oder mehrere der folgenden Informationen umfassen:
- eine Anzahl oder Anzeige der aufgetretenen Alarme (die von der Steuereinheit 20 erzeugt wurden),
- eine Mindesttemperatur der Heizmatte,
- eine Höchsttemperatur,
- eine Durchschnittstemperatur,
- eine Reihe von periodisch erfassten Heizmattentemperaturen,
- eine Anzahl von Abschaltungen der Steuereinheit 20,
- eine Anzahl von Abtrennungen der Heizmatte 10,
- eine Nutzungszeit,
- einen Identifikationscode für den durchgeführten Vorgang,
- einen Identifikationscode des betreuten Patienten und
- mindestens ein Vitalzeichen des betreuten Patienten.

10. Kit (30,31) für ein System (1) zur Unterkühlungsverhinderung, umfassend eine Heizmatte (10), die mit Heizmitteln versehen ist, und eine Steuereinheit (20), die angepasst ist, um den Betrieb der Heizmatte zu steuern, wobei das Kit Folgendes umfasst:
mindestens eine Abdeckung (30) für die Heizmatte (10) und ein abnehmbares Tag (31), wobei die Abdeckung (30) eine Folie umfasst, die geeignet ist, mindestens eine Auflagefläche der Heizmatte (10) abzudecken, auf der ein Patient beim Gebrauch liegt, und wobei
das abnehmbare Tag (31) eine elektronische Schaltung (311, 312) umfasst, die dazu angepasst ist:
ein Hochfrequenzantwortsignal als Reaktion auf den Empfang eines entsprechenden Hochfrequenztriggersignals zu übermitteln,
ein Aufzeichnungssignal zu empfangen, das Betriebsinformationen während der Nutzung des Systems (1) enthält, und
die im Aufzeichnungssignal enthaltene Betriebsinformation zu speichern, wobei die Betriebsinformationen umfassen:
- die Temperatur über einem vorgegebenen Grenzwert,
- eine Anzeige eines Zeitintervalls, in dem die Temperatur über dem vorgegebenen Grenzwert blieb,
- eine Anzeige eines Korrektureingriffs, um die Temperatur unter den vorgegebenen Grenzwert zu bringen, und
- vorzugsweise einen Zeitpunkt, zu dem die Korrektur durchgeführt wurde.

11. Kit (30, 31) nach Anspruch 10, wobei die elektronische Schaltung (311, 312) zum Speichern eines eindeutigen Tags oder Einheitsidentifikationscodes und vorzugsweise mindestens eines von Folgendem angepasst ist:
Statusinformationen, die anzeigen, ob die Abdeckung (30) genutzt wurde oder nicht, eine Anzeige einer Nutzungszeit der Abdeckung (30) und eine Anzeige einer Anzahl von Reaktivierungen der Steuereinheit (20) und/oder Wiederverbindungen der Steuereinheit (20) mit der Heizmatte (10), und
wobei das Hochfrequenzantwortsignal den eindeutigen Identifikationscode und vorzugsweise mindestens eine unter der Statusanzeige, der Nutzungszeitanzeige und der Anzahl von Reaktivierungen/Wiederverbindungen umfasst.

## Revendications

1. Système (1) de prévention de l'hypothermie comprenant :
un tapis chauffant (10) comprenant des moyens de chauffage et au moins un capteur de température,
une unité de commande (20), et
une housse jetable (30) pour le tapis chauffant (10),
où l'unité de commande (20) est reliée aux moyens de chauffage et à l'au moins un capteur de température du tapis chauffant (10) pour commander son fonctionnement afin d'amener le tapis chauffant (10) à une température souhaitée,
dans lequel
il comprend en outre une étiquette amovible (31) associée à la housse (30), ladite étiquette amovible (31) comprenant un circuit électronique (311, 312) adapté pour transmettre un signal de réponse à radiofréquence en réponse à la réception d'un signal de déclenchement à radiofréquence, et
en ce que
l'unité de commande (20) comprend une partie réceptrice (22) adaptée pour recevoir l'étiquette amovible (31) une fois séparée de la housse (30), et
en ce que
l'unité de commande est adaptée pour :
ne pas activer les moyens de chauffage du tapis (10) si elle ne reçoit pas le signal de réponse après avoir transmis le signal d'activation,
activer les moyens de chauffage du tapis (10) si elle reçoit le signal de réponse après avoir transmis le signal d'activation,
lorsqu'une température supérieure à une valeur limite prédéterminée est détectée, acquérir (4001) des informations de fonctionnement pendant l'utilisation du système (1), les informations de fonctionnement comprenant :
- la température supérieure à la valeur limite prédéterminée,
- une indication de l'intervalle de temps pendant lequel la température est restée supérieure à la valeur limite prédéterminée,
- une indication d'une intervention de correction pour ramener la température en dessous de la valeur limite prédéterminée, et
- de préférence, un instant donné lorsque la correction a été effectuée, et transmettre les informations de fonctionnement acquises dans au moins un signal d'enregistrement (4003), et
le circuit électronique (311, 312) de l'étiquette amovible (31) étant adapté pour :
recevoir ledit signal d'enregistrement, et
stocker les informations de fonctionnement contenues dans le signal d'enregistrement.

2. Système (1) selon la revendication 1, le signal de réponse transmis par le circuit électronique (311, 312) de l'étiquette amovible (31) contenant un code d'identification de l'étiquette, et l'unité de commande (20) étant en outre adaptée pour :
activer les moyens de chauffage du tapis (10) si le code d'identification de l'étiquette compris dans un signal de réponse nouvellement reçu n'est pas stocké dans un module de mémoire (24) compris dans l'unité de commande, et
stocker le code d'identification de l'étiquette compris dans le signal de réponse reçu dans le module de mémoire (24).

3. Système (1) selon la revendication 1, l'unité de commande (20) étant en outre configurée pour :
vérifier si le signal de réponse transmis par le circuit électronique (311, 312) de l'étiquette amovible (31) comprend un code d'identification de l'unité,
si le signal de réponse ne comprend pas de code d'identification d'unité, transmettre un signal de changement comprenant son propre code d'identification d'unité stocké dans un module de mémoire (24) compris dans l'unité de commande (20), et
ne pas activer les moyens de chauffage du tapis (10) si le code d'identification de l'unité compris dans un signal de réponse nouvellement reçu ne correspond pas à votre propre code d'identification d'unité.

4. Système (1) selon la revendication 2 ou 3, l'unité de commande (20) étant en outre adaptée pour :
déterminer une durée d'utilisation de la housse (30) sur la base des informations de durée d'utilisation stockées dans le module de mémoire (24) compris dans l'unité de commande (20) ou comprises dans le signal de réponse transmis par le circuit électronique (311, 312) de l'étiquette amovible (31),
vérifier le respect d'une durée limite d'utilisation sur la base de l'indication de durée d'utilisation, et
désactiver les moyens de chauffage du tapis (10) s'il est vérifié que ladite durée limite d'utilisation a été atteinte.

5. Système (1) selon la revendication 4, l'unité de commande (20) étant en outre adaptée pour :
stocker les informations de durée d'utilisation à partir d'un instant donné où ledit code d'identification est stocké, ou
transmettre un signal de début d'utilisation comprenant des informations de durée d'utilisation, et
le circuit électronique (311, 312) de l'étiquette amovible (31) étant adapté pour : recevoir le signal de début d'utilisation, et
stocker les informations de durée d'utilisation contenues dans ledit signal de début d'utilisation.

6. Système (1) selon l'une quelconque des revendications précédentes, le signal de réponse transmis par le circuit électronique (311, 312) de l'étiquette amovible (31) contenant des informations d'état,
l'unité de commande étant adaptée pour activer les moyens de chauffage du tapis (10) si les informations d'état n'indiquent pas une utilisation antérieure de la housse (30), et pour transmettre un signal de marquage après avoir reçu un signal de réponse, et
le circuit électronique (311, 312) de l'étiquette amovible (31) étant adapté pour : recevoir ledit signal de marquage, et
stocker des informations d'état indicatives d'une utilisation de la housse (30).

7. Système (1) selon l'une quelconque des revendications précédentes lorsqu'elles dépendent de la revendication 2 ou de la revendication 3, l'unité de commande (20) étant en outre adaptée pour :
déterminer un nombre de réactivations de l'unité de commande (20) et/ou de reconnexions de l'unité de commande (20) au tapis chauffant (10) sur la base des informations de redémarrage stockées dans le module de mémoire (24) compris dans l'unité de commande (20) et associées à un identifiant d'étiquette stocké correspondant ou compris dans le signal de réponse transmis par le circuit électronique (311, 312) de l'étiquette amovible (31), et
activer les moyens de chauffage du tapis (10) si ces informations de redémarrage indiquent un nombre de réactivations de l'unité de commande (20) et/ou de reconnexions de l'unité de commande (20) au tapis chauffant (10) inférieur à une valeur limite.

8. Système (1) selon la revendication 7, ladite indication de redémarrage étant un compteur de redémarrage, et
où, pour chaque code d'identification d'étiquette stocké, l'unité de commande est en outre adaptée pour :
stocker un compteur de redémarrage respectif, et
incrémenter le compteur de redémarrage associé à chaque fois qu'un signal de réponse comprenant le code d'identification d'étiquette correspondant est reçu, ou
l'unité de commande étant adaptée pour :
transmettre un signal d'incrémentation à chaque fois qu'elle reçoit un signal de réponse comprenant son code d'identification d'unité, et
le circuit électronique (311, 312) de l'étiquette amovible (31) étant adapté pour : stocker ledit compteur de redémarrage, et
incrémenter le compteur de redémarrage à chaque fois que le signal d'incrémentation est reçu.

9. Système (1) selon l'une quelconque des revendications précédentes, lesdites informations de fonctionnement comprenant un ou plusieurs éléments parmi :
- un nombre ou une indication des alarmes qui se sont produites (générées par l'unité de commande 20),
- une température minimale du tapis chauffant,
- une température maximale,
- une température moyenne,
- un ensemble de températures du tapis chauffant acquises périodiquement,
- un certain nombre d'arrêts de l'unité de commande 20,
- un certain nombre de déconnexions du tapis chauffant 10,
- une durée d'utilisation,
- un code d'identification de l'opération effectuée,
- un code d'identification du patient pris en charge, et
- au moins un signe vital du patient pris en charge.

10. Kit (30, 31) pour un système (1) de prévention de l'hypothermie comprenant un tapis chauffant (10) pourvu de moyens de chauffage, et d'une unité de commande (20) adaptée pour commander le fonctionnement du tapis chauffant, le kit comprenant :
au moins une housse (30) pour le tapis chauffant (10) et une étiquette amovible (31), ladite housse (30) comprenant un drap apte à recouvrir au moins une surface de support du tapis chauffant (10) sur laquelle un patient est allongé pendant l'utilisation, et dans lequel
l'étiquette amovible (31) comprend un circuit électronique (311, 312) adapté pour :
transmettre un signal de réponse à radiofréquence en réponse à la réception d'un signal de déclenchement à radiofréquence correspondant,
recevoir un signal d'enregistrement comprenant des informations de fonctionnement pendant l'utilisation du système (1), et
stocker les informations de fonctionnement contenues dans le signal d'enregistrement, les informations de fonctionnement comprenant :
- une température supérieure à une valeur limite prédéterminée,
- une indication de l'intervalle de temps pendant lequel la température est restée supérieure à la valeur limite prédéterminée,
- une indication d'une intervention de correction pour ramener la température en dessous de la valeur limite prédéterminée, et
- de préférence, un instant donné lorsque la correction a été effectuée.

11. Kit (30, 31) selon la revendication 10, le circuit électronique (311, 312) étant adapté pour stocker une étiquette unique ou un code d'identification d'unité et, de préférence, au moins l'une des informations suivantes : des informations d'état indiquant si la housse (30) a été utilisée ou non, une indication d'une durée d'utilisation de ladite housse (30) et une indication d'un nombre de réactivations de l'unité de commande (20) et/ou de reconnexions de l'unité de commande (20) au tapis chauffant (10), et
le signal de réponse à radiofréquence comprenant ledit code d'identification unique et, de préférence, au moins un élément parmi ladite indication d'état, ladite indication de durée d'utilisation et ledit nombre de réactivations/reconnexions.
